# EUROPEAN PATENT APPLICATION

(11) **EP 3 882 342 A1**
(43) Date of publication of application: **22.09.2021**
(21) Application number: 19885226.1
(22) Date of filing: 10.10.2019
(51) Int. Cl.: C12N 5/079, A61K 35/30, A61P 25/00, C12Q 1/06

(54) **METHOD FOR PRODUCING BRAIN ORGANOIDS**

(30) Priority: 15.11.2018 JP 2018214930
(71) Applicant: JSR Corporation, Tokyo 105-8640 (JP); Keio University, Tokyo, 108-8345 (JP)
(72) Inventor: HIRAMINE Hayato, Tokyo 105-8640 (JP); ISHIKAWA Mitsuru, Tokyo 160-8582 (JP); OKANO Hideyuki, Tokyo 160-8582 (JP)
(74) Representative: TBK
(86) International application number: PCT/JP2019/040001
(87) International publication number: WO 2020/100481

(57) **Abstract**

A method for producing a brain organoid is provided, including a step of culturing a neuroectoderm marker-positive cell aggregate in a medium containing an extracellular matrix with a concentration of more than 10% by volume.

## Description

### Technical Field

The present invention relates to a method for producing a brain organoid. More specifically, the present invention relates to a method for producing a brain organoid, a brain organoid, a kit for evaluating the drug efficacy of a test substance, a method for evaluating the drug efficacy of a test substance, a therapeutic agent for a disease based on a disorder of a nervous system cell or a nervous tissue, and a pharmaceutical composition for treating a disease based on a disorder of a nervous system cell or a nervous tissue. Priority is claimed on Japanese Patent Application No. 2018-214930, filed November 15, 2018, the content of which is incorporated herein by reference.

### Background Art

A mammalian cerebral cortex has a multi-layered structure (I-VI layer), which is gradually formed from a fetal cerebral cortex formation stage. The cerebral cortex is produced from a neuroepithelium of the dorsal telencephalon (mantle) and gradually abducts to form hemispherical brain vesicles on both sides. A posterior caudal side of the cerebral cortex is adjacent to the cortical hem, while a rostral side is adjacent to the lateral basal ganglia primordium (LGE, striatal primordium) and diaphragm via the paleocortex. In the multi-layered structure of an adult cerebral cortex, most is derived from adjacent tissues such as the cortical hem and diaphragm, and a layer I (original base in gestational period is referred to as marginal zone) of an outermost layer mainly formed from Reelin-positive Cajal-Retzius cells is present (in a case of the human cerebral cortex, some Reelin-positive cells are also produced directly from the cerebral cortex neuroepithelium). The remaining layers of the cortical plate have a characteristic pattern in which nerve cells are produced and disposed regularly in time and space. This is referred to as an inside-out pattern, in which deeper layers of nerve cells are produced faster from neural progenitor cells.

Unlike the cerebral cortex development of a mouse, of which much information is available, human cerebral cortex development is not understood in detail due to the limited use of human fetal brain tissue. So far, a three-dimensional culture method (SFEBq method) using mouse and human ES cells has been established, and it has been shown that this aggregate reproduces an initial process of cerebral cortex development. It has been reported that this method can also be applied to human iPS cells (Patent Document 1).

### Citation List

### Patent Document

[Patent Document 1]
PCT International Publication No. WO2015/076388

### Summary of Invention

### Technical Problem

An object of the present invention is to provide a method for producing a telencephalic marker-positive brain organoid.

### Solution to Problem

The present invention includes the following embodiments.
[1] A method for producing a brain organoid, including a step of culturing a neuroectoderm marker-positive cell aggregate in a medium containing an extracellular matrix with a concentration of more than 10% by volume.
[2] The method for producing a brain organoid according to [1], in which a concentration of the extracellular matrix in the medium is 20% by volume to 50% by volume.
[3] The method for producing a brain organoid according to [1] or [2], in which the medium further contains a Wnt signal enhancer.
[4] The method for producing a brain organoid according to any one of [1] to [3], in which the medium further contains a transforming growth factor β family signal transduction pathway inhibitor.
[5] The method for producing a brain organoid according to any one of [1] to [4], in which the step is a step of culturing the neuroectoderm marker-positive cell aggregate in a medium containing an extracellular matrix with a concentration of more than 10% by volume in a dispersed state without inserting the cell aggregate into the extracellular matrix.
[6] The method for producing a brain organoid according to any one of [1] to [5], further including a step of performing culture in a medium substantially not containing an extracellular matrix after the step.
[7] The method for producing a brain organoid according to [6], in which the step of performing culture in a medium substantially not containing an extracellular matrix is performed by suspension culture.
[8] The method for producing a brain organoid according to [6] or [7], in which the step of performing culture in a medium substantially not containing an extracellular matrix is performed under high oxygen partial pressure conditions.
[9] The method for producing a brain organoid according to any one of [6] to [8], in which the step of performing culture in a medium substantially not containing an extracellular matrix is performed while stirring.
[10] The method for producing a brain organoid according to any one of [1] to [9], in which the neuroectoderm marker-positive cell aggregate is derived from a human.
[11] A brain organoid produced by the production method according to any one of [1] to [10].
[12] The brain organoid according to [11], including at least a telencephalic marker-positive cell.
[13] The brain organoid according to [12], further including a telencephalic partial tissue marker-positive cell.
[14] The brain organoid according to [13], in which the telencephalic partial tissue marker-positive cell is at least one selected from the group consisting of a cerebral cortex, a basal ganglia, a hippocampus, and a choroid plexus.
[15] A kit for evaluating drug efficacy of a test substance, including the brain organoid according to any one of [11] to [14].
[16] A method for evaluating drug efficacy of a test substance, the method including a step of contacting the test substance with the brain organoid according to any one of [11] to [14] and a step of testing an effect of the test substance on the brain organoid.
[17] A therapeutic agent for a disease based on a disorder of a nervous system cell or a nervous tissue, the therapeutic agent including the brain organoid according to any one of [11] to [14].
[18] A pharmaceutical composition for treating a disease based on a disorder of a nervous system cell or a nervous tissue, the pharmaceutical composition containing the brain organoid according to any one of [11] to [14] as an effective component.

The present invention also includes the following embodiments.
[P1] A method for producing a brain organoid, including culturing a neuroectoderm marker-positive cell aggregate in a first medium containing more than 10% by volume of an extracellular matrix component, resulting in the formation of the brain organoid.
[P2] The method for producing a brain organoid according to [P1], in which a concentration of the extracellular matrix component in the first medium is 30% to 50% by volume.
[P3] The method for producing a brain organoid according to [P1] or [P2], in which the first medium further contains a Wnt signal enhancer.
[P4] The method for producing a brain organoid according to any one of [P1] to [P3], in which the first medium further contains a TGF-β family signal transduction pathway inhibitor.
[P5] The method for producing a brain organoid according to any one of [P1] to [P4], the method further including performing suspension culture on the brain organoid in a second medium.
[P6] The method for producing a brain organoid according to [P5], in which the second medium does not substantially contain an extracellular matrix component.
[P7] The method for producing a brain organoid according to [P5] or [P6], in which the suspension culture is performed under high oxygen partial pressure conditions.
[P8] The method for producing a brain organoid according to any one of [P5] to [P7], in which the suspension culture is performed by a stirring culture method.
[P9] The method for producing a brain organoid according to any one of [P1] to [P8], in which the neuroectoderm marker-positive cell aggregate is derived from a human.
[P10] The method for producing a brain organoid according to any one of [P1] to [P9], in which the brain organoid includes a forebrain, a telencephalon, or a telencephalic partial tissue.
[P11] The method for producing a brain organoid according to [P10], in which the telencephalic partial tissue is a cerebral cortex, a basal ganglia, a hippocampus, or a choroid plexus.
[P12] A brain organoid produced by the production method according to any one of [P1] to [P11].
[P13] A kit for evaluating toxicity and drug efficacy of a test substance, the kit including the brain organoid according to [P12].
[P14] A method for evaluating toxicity and drug efficacy of a test substance, the method including contacting the test substance with the brain organoid according to [P12] and testing an effect of the test substance on the brain organoid.
[P15] A therapeutic agent for a disease based on a disorder of a nervous system cell or a nervous tissue, the therapeutic agent including the brain organoid according to [P12].
[P16] A pharmaceutical composition for treating a disease based on a disorder of a nervous system cell or a nervous tissue, the pharmaceutical composition containing the brain organoid according to [P12] as an effective component.

### Advantageous Effects of Invention

According to the present invention, it is possible to provide a method for producing a telencephalic marker-positive brain organoid.

### Brief Description of Drawings

Fig. 1 is a photomicrograph showing results of Experimental Example 1.
Fig. 2 is a photomicrograph showing results of Experimental Example 1.
Fig. 3 is a photomicrograph showing results of bright-field observation of a cell aggregate on a 14th day of culture in Experimental Example 3.
Fig. 4 is a fluorescence photomicrograph showing results of immunostaining in Experimental Example 3.
Fig. 5 is a fluorescence photomicrograph showing results of immunostaining in Experimental Example 3.
Fig. 6 is a typical photomicrograph showing results of bright-field observation of a cell aggregate having different basal media in Experimental Examples 3 and 4.
Fig. 7 is a graph obtained by quantifying the results of Fig. 6.
Fig. 8 is a photomicrograph showing results of bright-field observation of a cell aggregate on a 40th day of culture in Experimental Example 5.
Fig. 9 is a fluorescence photomicrograph showing results of immunostaining of a brain organoid on the 40th day of culture in Experimental Example 5 (Matrigel concentration 50% by volume).
Fig. 10 is a fluorescence photomicrograph showing results of immunostaining of a brain organoid on the 40th day of culture in Experimental Example 5 (Matrigel concentration 30% by volume).
Fig. 11 is a fluorescence photomicrograph showing results of immunostaining of a brain organoid on the 40th day of culture in Experimental Example 5 (Matrigel concentration 10% by volume).
Fig. 12 is a fluorescence photomicrograph showing results of immunostaining of a brain organoid on the 40th day of culture in Experimental Example 5 (Matrigel concentration 2% by volume).
Fig. 13 is a fluorescence photomicrograph showing results of immunostaining of a brain organoid on a 70th day of culture in Experimental Example 5 (Matrigel concentration 50% by volume).
Fig. 14 is a fluorescence photomicrograph showing results of immunostaining of a brain organoid on the 70th day of culture in Experimental Example 5 (Matrigel concentration 30% by volume).
Fig. 15 is a fluorescence photomicrograph showing results of immunostaining of a brain organoid on the 70th day of culture in Experimental Example 5 (Matrigel concentration 10% by volume).

### Description of Embodiments

Hereinafter, the present invention will be described in more detail with reference to embodiments, but the present invention is not limited to the following embodiments.

### [Notation of gene name and protein name]

In the present specification, human genes and human proteins are represented by uppercase letters. In addition, the first letter of a mouse gene shall be represented by an uppercase letter, and subsequent letters shall be represented by a lowercase letter. In addition, a mouse protein shall be represented by an uppercase letter. However, in some cases, human genes, mouse genes, genes of other species, human proteins, mouse proteins, and proteins of other species may be described without strict distinction.

In the present specification, the notation representing a numerical value range such as "A to B" is synonymous with "A or more and B or less", and A and B are included in the numerical value range.

Unless otherwise specified, each substance exemplified in the present specification, for example, a substance contained in a medium or a substance used in each step, can be used alone or in combination of two or more.

In the present specification, "medium containing substance X" and "in the presence of substance X" mean a medium to which an exogenous substance X is added, a medium containing an exogenous substance X, or in the presence of an exogenous substance X. That is, in a case where a cell or tissue present in the medium expresses, secretes, or produces the substance X endogenously, an endogenous substance X is distinguished from the exogenous substance X, and a medium not containing an exogenous substance X does not fall under the category of "medium containing a substance X" even if the medium contains an endogenous substance X.

In the present specification, the term "cell aggregate" refers to a mass in which cells are adhered to each other. A cell mass, an embryoid body, a sphere, a spheroid, and a brain organoid are also included in the cell aggregate. In the cell aggregate, cells are surface-adhered to each other. Surface adhesion means that a ratio of the surface area of one cell adhered to the surface of another cell is, for example, 1% or more, preferably 3% or more, and more preferably 5% or more. The surface of the cell can be observed by staining with a reagent for staining a membrane (for example, DiI) or immunostaining with a cell adhesion factor (for example, E-cadherin or N-cadherin). In addition, it is also possible to quantify an adhesive region in the membrane. In the cell aggregate, there is a case where cell adhesion occurs such as a cell-cell junction and an adherence junction in a part or all of the cell aggregate.

### [Method for producing brain organoid]

For the production of a brain organoid, a method of inserting a neuroectoderm marker-positive cell aggregate into a gel-like Matrigel particle and performing suspension culture is widely known. However, since a gel-like Matrigel particle is easily broken at the time of insertion, a skillful technique is required to prevent breakage, and there is a problem in that the gel-like Matrigel particle is not suitable for mass production by an automated device. In addition, in a case where the neuroectoderm marker-positive cell aggregate cannot be inserted into the center of the gel-like Matrigel particle, the above-mentioned cell aggregate is detached from the gel-like Matrigel particle in suspension culture, and a brain organoid cannot be formed. In addition, since it takes time to form a solid Matrigel particle or to insert the above-mentioned cell aggregate, a method capable of more conveniently producing a brain organoid is sought. Therefore, the present embodiment provides a method for producing a brain organoid, including a step of culturing a neuroectoderm marker-positive cell aggregate in a medium containing an extracellular matrix with a concentration of more than 10% by volume (also referred to as "first medium") and preferably including a step of culturing the neuroectoderm marker-positive cell aggregate in a first medium containing an extracellular matrix with a concentration of more than 10% by volume in a dispersed state without inserting the cell aggregate into the extracellular matrix, resulting in formation of a brain organoid. As will be described later in the examples, according to the production method of the present embodiment, it is possible to conveniently produce a brain organoid.

In the present embodiment, brain organoid means a cell aggregate containing at least a telencephalic marker-positive cell. Examples of the telencephalic marker include, but are not limited to, FOXG1 (also referred to as BF1), SIX3, and the like. FOXG1 and SIX3 are also forebrain markers. The brain organoid of the present embodiment contains cells that express at least one telencephalic marker. In a preferred embodiment, the brain organoid is a cell aggregate containing FOXG1-positive cells. It is also possible to prepare sections of a brain organoid, to perform immunostaining with 4', 6-diamidino-2-phenylindole (DAPI) and anti-FOXGl antibody, and to calculate a proportion of FOXG1-positive cells in the brain organoid. As for the brain organoid, the number of cells contained in the brain organoid is 50% or more, preferably 70% or more, more preferably 90% or more, and the brain organoid is preferably telencephalic marker-positive.

The brain organoid produced by the production method of the present embodiment preferably contains at least one selected from a forebrain marker-positive cell and a telencephalic partial tissue marker-positive cell, in addition to a telencephalic marker-positive cell. Examples of the telencephalic partial tissue marker-positive cell include a telencephalic partial tissue marker-positive cell of the cerebral cortex, the basal ganglia, the hippocampus, the choroid plexus, and the like. Whether or not the brain organoid contains a forebrain marker-positive cell and a telencephalic partial tissue marker-positive cell, in addition to a telencephalic marker-positive cell, can be morphologically determined. Alternatively, the expression of a marker gene or marker protein characteristic of each cell can be measured and determined.

Examples of the forebrain marker include FOXG1, SIX3, and the like. In addition, examples of the cerebral cortex marker include PAX6, which is a neural stem cell and neural progenitor cell marker, CTIP2, which is a layer V marker of the cerebral cortex, SATB2, which is a layer II/III marker of the cerebral cortex, and the like. In addition, examples of the basal ganglia marker include NKX2.1, GSH2, and the like. In addition, examples of the hippocampal marker include KA1, ZBTB2, and the like. In addition, examples of the choroid plexus marker include TTR, LMX1A, and the like.

The neuroectoderm marker-positive cell aggregate is preferably induced from a stem cell, and the stem cell is preferably a pluripotent stem cell or induced from a pluripotent stem cell.

### «Pluripotent stem cell»

A pluripotent stem cell is a stem cell that can be cultured in vitro and has the ability to be differentiated into all three germ layers (ectoderm, mesoderm, endoderm) and/or all cell lineages belonging to extraembryonic tissues (pluripotency).

The pluripotent stem cell may be a non-genetically modified pluripotent stem cell or a genetically modified pluripotent stem cell. Since a brain organoid obtained from genetically modified pluripotent stem cells can be used as a model of a brain having a brain disease such as neurodegeneration, the brain organoid can be suitably used in a method for evaluating the toxicity or drug efficacy of a test substance of the present embodiment, for example.

The pluripotent stem cells can be induced from fertilized eggs, cloned embryos, reproductive stem cells, tissue stem cells, somatic cells, and the like. Examples of the pluripotent stem cell include an embryonic stem cell (ES cell), an embryonic germ cell (EG cell), an artificial pluripotent stem cell (induced pluripotent stem cell (iPS cell), and the like.

The pluripotent stem cells also include a multi-lineage differentiating stress enduring cell (Muse cell) obtained from a mesenchymal stem cell (MSC) and an embryonic germ cell (EG cell, embryonic reproduction stem cell) established from a primordial germ cell.

ES cells were first established in 1981 and have been applied to the production of knockout mice since 1989. Human ES cells were established in 1998 and are being used for regenerative medicine. The ES cells can be produced by culturing the inner cell mass on feeder cells or in a medium containing Leukemia Inhibitory Factor (LIF). A method for producing the ES cells is described in WO96/22362, WO02/101057, US5,843,780, US6,200,806, US6,280,718, and the like. The ES cells can be obtained from a predetermined institution, and commercially available products can be purchased. For example, KhES-1, KhES-2, and KhES-3, which are human ES cells, are available from the Institute for Frontier Life and Medical Sciences, Kyoto University.

Rx :: GFP strain (derived from KhES-1), which is a human ES cell, is available from RIKEN, the Institute of Physical and Chemical Research. In addition, EB5 cells, which are mouse ES cells, are available from RIKEN, the institute of Physical and Chemical Research. In addition, the D3 strain, which is a mouse ES cell, is available from ATCC.

A nuclear transplanted ES cell (ntES cell), which is one of the ES cells, can be established from cloned embryos produced by transplanting the cell nuclei of somatic cells into an egg from which the cell nuclei have been removed.

The EG cells can be produced by culturing primordial germ cells in a medium containing mSCF, LIF and bFGF (for example, refer to Matsui Y., et al., Derivation of pluripotential embryonic stem cells from murine primordial germ cells in culture, Cell, 70 (5), 841-847, 1992).

In the present embodiment, "iPS cell" means a cell in which pluripotency is induced by reprogramming a somatic cell by a known method and the like. Specifically, examples of the cell include a cell obtained by reprogramming a differentiated somatic cell such as fibroblasts, peripheral blood mononuclear cells, and lymphocytes by expression of any combination of a plurality of genes selected from a reprogramming gene group including Oct3/4, Sox2, Klf4, Myc (c-Myc, N-Myc, L-Myc), Glis1, Nanog, Sal14, Lin28, Esrrb, and the like, and inducing pluripotency.

Examples of preferred combinations of reprogramming gene groups include a combination of (1) Oct3/4, Sox2, Klf4, and Myc (c-Myc or L-Myc), or a combination of (2) Oct3/4, Sox2, Klf4, Lin28, and L-Myc (refer to Okita K., et al., An efficient nonviral method to generate integration-free human-induced pluripotent stem cells from cord blood and peripheral blood cells, Stem Cells, 31 (3), 458-466, 2013).

iPS cells were established in mouse cells by Yamanaka et al. in 2006 (Takahashi K. and Yamanaka S., Induction of pluripotent stem cells from mouse embryonic and adult fibroblast cultures by defined factors, Cell, 126 (4), 663- 676, 2006). iPS cells were also established in human fibroblasts in 2007 and have pluripotency and self-renewal ability similar to those of ES cells (for example, refer to Takahashi K., et al., Induction of pluripotent stem cells from adult human fibroblasts by defined factors, Cell, 131 (5), 861-872, 2007; Yu J., et al., Induced Pluripotent Stem Cell Lines Derived from Human Somatic Cells, Science, 318 (5858), 1917-1920, 2007; Nakagawa M., et al., Generation of induced pluripotent stem cells without Myc from mouse and human fibroblasts, Nat Biotechnol, 26 (1), 101-106, 2008 and the like).

In addition to the method for producing iPS cells from somatic cells by reprogramming by gene expression, iPS cells can also be induced from somatic cells by addition of a compound and the like (Hou P., et al., Pluripotent stem cells induced from mouse somatic cells by small-molecule compounds, Science, 341 (6146), 651-654, 2013).

In the present embodiment, it is also possible to use the strained iPS cells. For example, human iPS cell strains such as 201B7 cells, 201B7-Ff cells, 253G1 cells, 253G4 cells, 1201C1 cells, 1205D1 cells, 1210B2 cells, and 1231A3 cells established at Kyoto University are available from Kyoto University and iPS Academia Japan Co., Ltd. In addition, examples of the strained iPS cells include PCPhiPS771 sold by ReproCELL Inc. In addition, examples of the strained iPS cells include XFiPS-F44-3F-2 established by Institute of Health Carlos III.

Examples of the somatic cells used at the time of producing the iPS cells include tissue-derived fibroblasts, blood cells (for example, peripheral blood mononuclear cells or T cells), hepatocytes, pancreatic cells, intestinal epithelial cells, smooth muscle cells, and the like.

In a case of reprogramming by expression of several kinds of genes at the time of producing iPS cells, means for expressing the genes is not particularly limited. Examples of the means include an infection method using a viral vector (for example, a retrovirus vector, a lentivirus vector, a Sendai virus vector, an adenovirus vector, and an adeno-associated virus vector), a gene introduction method (for example, calcium phosphate method, lipofection method, retronectin method, and electroporation method) using a plasmid vector (for example, a plasmid vector and an episomal vector), a gene introduction method using an RNA vector (for example, calcium phosphate method, lipofection method, and electroporation method), a method of directly injecting a protein or mRNA, and the like.

The pluripotent stem cells used in the production method of the present embodiment are preferably ES cells or iPS cells, and more preferably iPS cells.

Genetically modified pluripotent stem cells can be conveniently prepared by transfecting pluripotent stem cells with artificial nucleases such as ZFN, TALEN, and CRISPR. In the artificial nuclease, a double-strand DNA break (DSB: double strand break) is introduced into a target gene, an insertion/deletion mutation is introduced by non-homologous end joining (NHEJ), which is one of the DSB repair mechanisms, and a genetically modified cell strain in which the target gene is disrupted, that is, a knockout cell strain is prepared. Since it is possible to prepare a knockout cell strain in a shorter period of time at a lower cost in a more efficient manner than a gene modification technique in the related art, the technique has become widely used as a technique for preparing a gene-modified cell (for example, refer to Hsu PD et al., Development and Applications of CRISPR-Cas9 for Genome Engineering, Cell, 157 (6), 1262-1278, 2014 and the like).

Attempts have also been made to knock in a gene such as GFP into a target genome region (or gene) using an artificial nuclease. A donor plasmid having a homologous sequence of about 500 bp-1 kbp in the target genome region is used at both ends of a knock-in sequence such as GFP. By introducing a donor plasmid into pluripotent stem cells together with the artificial nuclease, the artificial nuclease introduces DSB into a target sequence and a gene such as GFP knocks in the target sequence by homologous recombination (HR), which is another DSB repair mechanism, using a homologous sequence of the donor plasmid.

In addition, as a method that does not use a donor plasmid, by using single-stranded DNA (single-stranded oligodexynucleotides: ssODN), single nucleotide substitutions of target genes or short DNA sequences of several tens of bp or less such as His tags and LoxP can be introduced. With a base sequence to be introduced interposed, by artificially synthesizing ssODN containing a homologous sequence of 40 to 60 bp at both ends and introducing into a fertilized egg together with an artificial nuclease, it is possible to conveniently and efficiently prepare a knock-in cell strain using single-strand annealing (SSA), which is a highly efficient DSB repair mechanism.

At the time of performing knock-in using a donor plasmid, it is necessary to add a homologous sequence of the target genome region to the plasmid containing the gene to be knocked in. The homologous sequence is amplified by PCR and the like and cloned with ligation and Escherichia coli to prepare a donor plasmid. In addition, as a method for selecting a knock-in cell strain, methods such as positive selection, promoter selection, negative selection, and poly A selection can be used. Examples of the method for selecting a target homologous recombinant from the selected cell strains include a Southern hybridization method for genome DNA, a PCR method, and the like.

The pluripotent stem cells are preferably mammal-derived pluripotent stem cells, the mammals are preferably rodents and primates, more preferably primates, and the primates are preferably humans.

"Mammals" include rodents, ungulates, carnivora, primates, and the like. Rodents include mice, rats, hamsters, guinea pigs, and the like. Ungulates includes pigs, cows, goats, horses, sheep, and the like. The order Carnivora includes dogs, cats, and the like. Primates refers to mammals belonging to the order Primates, and examples of primates include the suborder Prosimii such as fox monkeys, Loris, and tree shrews, and Anthropoidea such as monkeys, apes, and humans.

### «Proliferation of pluripotent stem cells»

In the method for producing a brain organoid of the present embodiment, pluripotent stem cells can be proliferated and used. At the time when pluripotent stem cells are proliferated, the pluripotent stem cells can be proliferated in the presence of feeder cells or in the absence of feeder cells (feeder-free).

At the time of proliferating iPS cells in the presence of feeder cells, iPS cells can be proliferated in the presence of undifferentiated maintenance factors by a known method. As the proliferation medium used at the time of proliferating iPS cells in the absence of feeder cells, for example, a known ES cell and/or iPS cell maintenance medium, or a feeder-free medium for establishing iPS cells can be used. Examples of the feeder-free medium for establishing iPS cells in a feeder-free manner include Essential 8 medium, TeSR medium, mTeSR medium, mTeSR-E8 medium (all manufactured by StemCell Technologies), StemFit medium (manufactured by Ajinomoto Co., Inc.), and the like.

As an incubator used for proliferating pluripotent stem cells, if it is possible to "adhesively culture", it is possible to appropriately select an incubator according to the scale of culture, culture conditions, and culture period. Examples of such an incubator include a flask, a tissue culture flask, a culture dish (dish), a tissue culture dish, a multi-dish, a microplate, a microwell plate, a multi-plate, a multi-well plate, a chamber slide, a petri dish, a tube, a tray, a culture bag, a microcarrier, a bead, a stack plate, a spinner flask, or a roller bottle.

These incubators are preferably cell-adhesive in order to enable adhesion culture. Examples of the cell-adhesive incubator include an incubator in which the surface of the incubator is artificially treated for the purpose of improving adhesion to cells, specifically, a surface-processed incubator or an incubator of which the inside is coated with a coating agent. Examples of the coating agent include laminin (laminin α5β1γ1 (hereinafter, referred to as "laminin 511" in some cases), laminin α1β1γ1 (hereinafter, referred to as "laminin 111" in some cases), laminin fragment (laminin 511E8 and the like), and the like), extracellular matrix such as entactin, collagen, gelatin, Vitronectin, synthemax (Corning Inc.), and Matrigel; polymers such as polylysine and polyornithine, and the like. Examples of the surface-processed incubator include a surface-processed culture container such as a positive charge treatment.

For the proliferation of pluripotent stem cells, it is preferable to add a ROCK (Rho-associated coiled-coil forming kinase/Rho-associated kinase) inhibitor to the medium and culture the cells. By adding a ROCK inhibitor, it is possible to suppress cell death during cell dispersal of pluripotent stem cells, particularly human iPS/ES cells. In a case where the cells are exfoliated, it is preferable to appropriately add a ROCK inhibitor after exfoliation and culture thereof. In a case where the ROCK inhibitor is added, culture may be performed for at least one day after the addition, and it is preferable to remove the ROCK inhibitor after the culture. In addition, for the proliferation of pluripotent stem cells, it is possible to perform culture in a medium containing a ROCK inhibitor, more than one day before the pluripotent stem cells are exfoliated from the medium, and preferably one day before.

The ROCK inhibitor is not particularly limited as long as it can suppress the function of Rho kinase (ROCK), and examples thereof include Y-27632 (for example, refer to Ishizaki T., et al., Pharmacological properties of Y-27632, a specific inhibitor of rho-associated kinases, Mol Pharmacol, 57 (5), 976-983, 2000; Narumiya S., et al., Use and properties of ROCK-specific inhibitor Y-27632, Methods Enzymol 325, 273-284, 2000 and the like), Fasudil/HA1077 (for example, refer to Uehata M., et al., Calcium sensitization of smooth muscle mediated by a Rho-associated protein kinase in hypertension, Nature, 389 (6654), 990-994, 1997), H-1152 (for example, refer to Sasaki Y., et al., The novel and specific Rho-kinase inhibitor (S)-(+)-2-methyl-1-[(4-methyl-5-isoquinoline) sulfonyl]-homopiperazine as a probing molecule for Rho-kinase-involved pathway, Pharmacol Ther., 93 (2-3), 225-232, 2002.), Wf-536 (for example, refer to Nakajima M., et al., Effect of Wf-536, a novel ROCK inhibitor, against metastasis of B16 melanoma, Cancer Chemotherapy and Pharmacology, 52 (4), 319-324, 2003), and derivatives thereof, and antisense nucleic acids against ROCK, RNA interference-inducing nucleic acids (for example, siRNA), dominant negative variants, and expression vectors thereof.

### <<Neuroectoderm marker-positive cell aggregate>>

Subsequently, the proliferated polyfunctional stem cells are cultured to produce a neuroectoderm marker-positive cell aggregate.

A medium used for culturing a neuroectoderm marker-positive cell aggregate (hereinafter, referred to as "aggregation medium") will be described.

As a basal medium of the aggregation medium, a medium usually used for culturing animal cells can be used. Examples of the basal medium include a medium capable of being used for culture of animal cells, such as BME medium, BGJb medium, CMRL 1066 medium, Glasgow MEM (GMEM) medium, Improved MEM Zinc Option medium, IMDM medium, Medium 199 medium, Eagle MEM medium, αMEM medium, DMEM medium, F-12 medium, DMEM/F12 medium, IMDM/F12 medium, Ham's medium, RPMI 1640 medium, Fischer's medium, or a mixed medium thereof.

In culturing a neuroectoderm marker-positive cell aggregate, a serum-free medium can also be used. "Serum-free medium" means a medium that does not contain unadjusted or unpurified serum. A medium in which purified blood-derived components or animal tissue-derived components (for example, proliferation factors) are mixed is also included in the serum-free medium unless the medium contains unadjusted or unpurified serum. The serum-free medium may contain a serum substitute. Examples of the serum substitute include those appropriately containing albumin, transferrin, fatty acid, collagen precursor, trace element, 2-mercaptoethanol, or thioglycerol, and equivalents thereof.

The serum substitute can be prepared by the method described in WO98/30679, for example. A commercially available product may be used as a serum substitute. Commercially available serum substitutes include Knockout (TM) Serum Replacement (manufactured by Thermo Fisher Scientific Inc.: hereinafter, referred to as "KSR"), Chemically-defined Lipid concentrated (manufactured by Thermo Fisher Scientific Inc.), Glutamax (TM) (manufactured by Thermo Fisher Scientific Inc.), B27 (manufactured by Thermo Fisher Scientific Inc.), N2 supplement (manufactured by Thermo Fisher Scientific Inc.), 1 × Non-essential Amino Acids (manufactured by Thermo Fisher Scientific Inc.), and the like.

The aggregation medium preferably contains a bone morphogenetic protein (BMP) signal transduction pathway inhibitor or a transforming growth factor-β (TGF-β) family signal transduction pathway inhibitor.

The BMP signal transduction pathway inhibitor is not particularly limited as long as it is a substance that inhibits the signal transduction pathway caused by BMP, and may be any of nucleic acids, proteins, and low-molecular-weight organic compounds. Here, examples of the BMP include BMP2, BMP4, BMP7, GDF7, and the like.

Examples of the BMP signal transduction pathway inhibitors include substances that directly act on BMP (Noggin protein, antibody, aptamer, and the like), substances that suppress the expression of genes encoding BMP (antisense oligonucleotides, siRNA and the like), substances that inhibit binding of a BMP receptor (BMPR) and BMP, substances that inhibit the physiological activity caused by signal transduction by the BMP receptor, and the like. Examples of BMPR include ALK2, ALK3, and the like.

Examples of the BMP signal transduction pathway inhibitor include LDN193189, Dorsomorphin, and the like. LDN193189 (4-[6-(4-piperazine-1-ylphenyl) pyrazolo [1,5-a] pyrimidine-3-yl] quinoline) is a BMPR (ALK2/3) inhibitor (hereinafter, BMPR inhibitor), and is usually commercially available in the form of hydrochloride. In addition, a protein known as a BMP signal transduction pathway inhibitor (Chordin, Noggin, or the like) may be used.

The BMP signal transduction pathway inhibitor is preferably Dorsomorphin.

A final concentration of the BMP signal transduction pathway inhibitor contained in the aggregation medium is preferably 0.5 to 10 µM, more preferably 0.75 to 5 µM, and further more preferably 1 to 3 µM. The range of the above numerical values is preferable since the nerve cells forming the layer structure in the brain organoid are spatially and regularly disposed.

The TGF-β family signal transduction pathway inhibitor represents a substance that inhibits the TGF-β family signal transduction pathway, that is, the signal transduction pathway transmitted by the Smad family, and specific examples thereof can include a TGF-β signal transduction pathway inhibitor, Nodal/Activin signal transduction pathway inhibitor, and the like.

The TGF-β signal transduction pathway inhibitor is not particularly limited as long as it is a substance that inhibits the signal transduction pathway caused by TGF-β, and may be any of nucleic acids, proteins, and low-molecular-weight organic compounds.

Examples of the TGF-β signal transduction pathway inhibitors include substances that directly act on TGF-β (for example, proteins, antibodies, aptamers, and the like), substances that suppress the expression of genes encoding TGF-β (for example, antisense oligonucleotides, SiRNA, and the like), substances that inhibit the binding of TGF-β receptor and TGF-β, substances that inhibit physiological activity caused by signal transduction by TGF-β receptor (for example, inhibitors of TGF-β receptor, inhibitors of Smad, and the like), and the like.

Examples of the protein known as TGF-β signal transduction pathway inhibitors include Lefty and the like. As the TGF-β signal transduction pathway inhibitor, a compound known to those skilled in the art can be used, and specific examples thereof include SB431542, LY-364947, SB-505124, A-83-01, and the like. Here, SB431542 (4-(5-benzol [1,3] dioxol-5-yl-4-pyridine-2-yl-1H-imidazole-2-yl)-benzamide) and A-83-01 (3-(6-methyl-2-pyridinyl)-N-phenyl-4-(4-quinolinyl)-1H-pyrazole-1-carbothioamide) are compounds known as inhibitors (that is, TGF-β R inhibitors) of TGF-β receptor (ALK5) and Activin receptor (ALK4/7). The TGF-β signal transduction pathway inhibitor is preferably SB431542 or A-83-01, and A-83-01 is preferable since it facilitates differentiation into the initial telencephalon.

The final concentration of the TGF-β signal transduction pathway inhibitor contained in the aggregation medium is preferably 0.5 to 10 µM, more preferably 0.75 to 5 µM, and further more preferably 1 to 3 µM.

In a case where the BMP signal transduction pathway inhibitor is Dorsomorphin and the TGF-β signal transduction pathway inhibitor is A-83-01 as the aggregation medium, a period of induction from pluripotent stem cells to neuroectoderm marker-positive cell aggregates is preferably 3 to 14 days, more preferably 4 to 12 days, and further more preferably 5 to 10 days. It is preferable that the value be within the above numerical value range since the efficiency of inducing brain vesicles containing neuroepithelial cells is improved.

As a method of culturing in the aggregation medium, suspension culture is preferable, and suspension culture is preferably performed while stirring the aggregation medium. Suspension culture refers to culturing while maintaining a state in which cell aggregates are suspended and present in a culture solution, and a method of performing the culture. The suspension culture is performed under conditions where cells or cell aggregates are not adhered to culture equipment or the like, and culture (adhesion culture or adhesion culture method) performed under conditions where cells or cell aggregates are adhered to culture equipment or the like is not included in the category of suspension culture. In this case, the adhesion of cells means that a strong cell-substratum junction can be formed between the cells or cell aggregates and the culture equipment. More specifically, suspension culture refers to culture under conditions in which a strong cell-substratum junction is not formed between cells or cell aggregates and culture equipment, and adhesion culture refers to culture under conditions in which a strong cell-substratum junction is formed between cells or cell aggregates and culture equipment.

At the time of culturing the pluripotent stem cells in the aggregation medium, the pluripotent stem cells can be dispersed. By dispersing pluripotent stem cells, it is possible to obtain neuroectoderm marker-positive cell aggregates excellent in uniformity. Dispersion means that cells and tissues are separated into small cell fragments (2 cells or more and 100 cells or less, preferably 50 cells or less) or single cells by dispersion treatment such as enzyme treatment or physical treatment. A certain number of dispersed cells means a collection of a certain number of cell fragments or single cells. Examples of the method for dispersing pluripotent stem cells include mechanical dispersion treatment, cell dispersion treatment, cell protective agent addition treatment, and the like. These treatments may be combined. Preferably, the cell dispersion treatment is performed, and subsequently the mechanical dispersion treatment may be performed. Examples of the method of mechanical dispersion treatment include pipetting treatment and a scraping operation with a scraper.

Examples of the cell dispersion used for the cell dispersion treatment include a solution containing any of enzymes such as trypsin, collagenase, hyaluronidase, elastase, pronase, DNase, and papain, and a chelating agent such as ethylene diamine tetraacetic acid. As a commercially available cell dispersion, for example, TrypLE Select (manufactured by Thermo Fisher Scientific Inc.) or TrypLE Express (manufactured by Thermo Fisher Scientific Inc.) can also be used.

At the time of dispersing the pluripotent stem cells, the cell death of the pluripotent stem cells may be suppressed by treating the pluripotent stem cells with a cell protective agent. Examples of the cell protective agent used for the cell protective agent treatment include fibroblast cell proliferation factor (fibroblast growth factor (FGF)) signal transduction pathway activator, heparin, and insulin-like growth factor (IGF) signal transduction pathway activator, serum, serum substitutes, and the like.

In order to suppress the cell death induced by the dispersion (particularly, the cell death of human pluripotent stem cells), a ROCK inhibitor or a Myosin inhibitor may be added at the time of dispersion. Examples of the ROCK inhibitor include Y-27632, Fasudil (HA1077), H-1152, and the like. Examples of the Myosin inhibitor include Blebbistatin. Preferred cell protective agents include ROCK inhibitors.

In culturing neuroectoderm marker-positive cell aggregates, it is preferable to rapidly collect dispersed pluripotent stem cells in a narrow space and culture thereof. In a case of such culture, an epithelial-like structure can be reproducibly formed in the brain organoid that is induced to differentiate from the formed cell aggregates. Examples of the culture plate having a narrow space include a plate having a narrow well (for example, a plate having a well bottom area of about 0.1 to 2.0 cm² in terms of a flat bottom), a micropore, a small centrifuge tube, and the like.

Examples of the plate having a narrow well include a 24-well plate (area is about 1.88 cm² in terms of flat bottom), a 48-well plate (area is about 1.0 cm² in terms of flat bottom), a 96-well plate (area is about 0.3 cm² in terms of flat bottom, inner diameter is about 6 to 8 mm), a 384-well plate, and the like. Among these, a 96-well plate is preferable.

As the shape of the plate having a narrow well, examples of the shape of the bottom surface when the well is viewed from above include polygons, rectangles, ellipses, perfect circles, and the like, and perfect circles are preferable. As the shape of the plate having a narrow well, the shape of the bottom surface when the well is viewed from the side may be a flat bottom structure, or a structure in which an outer peripheral portion is high and an inner recess is low. Examples of the shape of the bottom surface include a U bottom, a V bottom, and an M bottom, preferably a U bottom or a V bottom, and more preferably a V bottom. As a plate having a narrow well, a plate having an unevenness or a dent on the bottom surface of a cell culture dish (for example, a dish of 60 to 150 mm, a culture flask) may be used. As the bottom surface of the plate having a narrow well, it is preferable to use a cell non-adhesive bottom surface, preferably a cell non-adhesive coated bottom surface.

Whether cell aggregates are formed and the uniformity thereof can be determined based on the size and number of cells of the cell aggregates, macroscopic morphology, microscopic morphology by tissue staining analysis and the uniformity, and the like. In addition, whether epithelial-like structures in the cell aggregates are formed and the uniformity thereof can be determined based on the macroscopic morphology of the cell aggregates, the microscopic morphology by tissue staining analysis and the uniformity, the expression of differentiated and undifferentiated markers and the uniformity, expression control of the differentiated marker and the synchrony, the reproducibility of the differentiation efficiency between cell aggregates, and the like.

Whether a neuroectoderm marker-positive cell aggregate is formed can be determined by the neuroectoderm marker being positive. Examples of the neuroectoderm marker include NESTIN, βIII-TUBULIN, and the like.

### «Culture of neuroectoderm marker-positive cell aggregate»

The production method of the present embodiment includes a step of culturing a neuroectoderm marker-positive cell aggregate in a first medium containing an extracellular matrix with a concentration of more than 10% by volume. Preferably, the production method of the present embodiment includes a step of culturing a neuroectoderm marker-positive cell aggregate in the first medium containing the extracellular matrix with a concentration of more than 10% by volume in a dispersed state without inserting into the extracellular matrix. A brain organoid is formed by culturing the neuroectoderm marker-positive cell aggregate in the first medium.

The "extracellular matrix component" refers to various components usually found in the extracellular matrix. In the present embodiment, it is preferable to use a basal membrane component. Examples of the basal membrane component include type IV collagen, laminin, heparan sulfate proteoglycan, entactin, nidogen, and the like. Commercially available extracellular matrix components can be used as the extracellular matrix component to be added to the medium, and examples thereof include Matrigel (manufactured by Corning Inc.), human laminin (manufactured by Merck KGaA), and the like. Matrigel is a basal membrane preparation derived from Engelbreth Holm Swarn (EHS) mouse sarcoma. The main components of Matrigel are type IV collagen, laminin, heparan sulfate proteoglycan, entactin, and nidogen, but in addition to these, TGF-β, FGF, tissue plasminogen activator, and proliferation factor naturally produced by EHS tumors are included. Matrigel's growth factor reduced product has a lower concentration of proliferation factors than normal Matrigel, and the standard concentration is epidermal growth factor (EGF) < 0.5 ng/mL, nerve growth factor (NGF) < 0.2 ng/mL, platelet-derived growth factor (PDGF) < 5 pg/mL, IGF-1 ≤ 5 ng/mL, and TGF-β ≤ 1.7 ng/mL. As the extracellular matrix component, it is preferable to use Matrigel's growth factor reduced product.

The concentration of extracellular matrix components in the first medium contributes to the formation of neural tube-like structures. In addition, in the construction of the cerebral cortex-like hierarchical structure in long-term culture, it also affects the production time of upper layer nerve cells and the thickness of the upper layer.

In a case where Matrigel is used as an extracellular matrix component, Matrigel and the first medium are mixed by pipetting on an ice bath. When pipetting is performed 15 times or more, Matrigel is dispersed in the medium. Dispersion means a state in which a mass of extracellular matrix cannot be visually observed in the medium. When Matrigel cannot be dispersed in the medium, aggregation occurs between the obtained brain organoid.

Assuming that the combined volume of the first medium and the extracellular matrix is 100% by volume, an upper limit of the concentration of the extracellular matrix is preferably 70% by volume, more preferably 60% by volume, and further more preferably 50% by volume. On the other hand, a lower limit of the concentration of the extracellular matrix is preferably 10% by volume, more preferably 20% by volume, and further more preferably 30% by volume. When the concentration is 10% by volume or less, in a case of performing immunostaining of the brain organoid section on the 70th day of the culture, expression of SATB2, which is a layer II/III marker of the cerebral cortex, is hardly observed. In addition, when the concentration is within a range of 30% to 50% by volume, the expression of SATB2, which is a layer II/III marker of the cerebral cortex, is clearly observed in the brain organoid section on the 70th day of the culture. In addition, when the concentration is within the range of 30% to 50% by volume, a period until SATB2 is expressed is preferably short.

The first medium can be prepared using a medium used for culturing animal cells as a basal medium. The basal medium is not particularly limited as long as the basal medium is a medium that can be used for culture of animal cells, such as BME medium, BGJb medium, CMRL1066 medium, Glasgow MEM medium, Improved MEM Zinc Option medium, IMDM medium, Medium 199 medium, Eagle MEM medium, αMEM medium, DMEM medium, Ham's medium, Ham's F-12 medium, RPM11640 medium, Fischer's medium, a mixed medium thereof, and the like.

The first medium can contain an N2 supplement (product name manufactured by Thermo Fisher Scientific Inc.), chemically defined lipid concentrate, serum, serum substitute, heparin, and the like. The serum substitutes are the same as those described above.

The production method of the present embodiment may include a step of culturing a neuroectoderm marker-positive cell aggregate in a first medium, and can have another step not using the first medium. For example, after performing a step of culturing a neuroectoderm marker-positive cell aggregate using a medium other than the first medium (a medium containing N2 supplement and chemically defined lipid concentrate and not containing serum, heparin, and extracellular matrix component), a step of performing culture in the first medium can be carried out by changing the medium to the first medium during the step (for example, after the stage where a hemispherical neural tube-like structure having ventricular cavities in a Foxg1-positive cell aggregate is formed).

The first medium may further contain a Wnt signal enhancer. Examples of the Wnt signal enhancer include Wnt agonist such as Wnt protein, GSK-3β inhibitor, and R-Spondin, Dkk (inhibitor of Wnt inhibitory protein), and the like. Among these, as the Wnt signal enhancer, Wnt protein and GSK-3β inhibitor are preferable.

Examples of the GSK-3β inhibitor include CHIR99021 (6-[[2-[[4- (2,4-Dichlopheneyl)-5-(5-methyl-1H-imidazol-2-yl)-2-pyrimidinyl] amino] ethyl] amino]-3-pyridinecarbonitrile), Kenpaullone, 6-Bromoindirubin-3'-oxime (BIO), and the like. Among these, CHIR99021 is preferable as the GSK-3β inhibitor.

As the Wnt protein, Wnt proteins derived from various organisms can be used. Among the Wnt proteins derived from organisms, Wnt proteins derived from mammals are preferable. Examples of mammalian Wnt proteins include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, Wnt16, and the like. Among these, Wnt3a is preferable, and Wnt3a is more preferably a complex with afamin.

In a case where CHIR99021 is used as the Wnt signal enhancer, the final concentration contained in the medium is preferably 0.1 µM to 30 µM, more preferably 0.5 µM to 10 µM, and further more preferably 1 µM to 5 µM. It is preferable that the value be within the above numerical value range since the efficiency of inducing brain vesicles containing neuroepithelial cells is improved.

In a case where Wnt3a is used as the Wnt signal enhancer, the final concentration contained in the medium is preferably 0.1 ng/mL to 20 ng/mL, more preferably 0.5 ng/mL to 15 ng/mL, and further more preferably 1 ng/mL to 10 ng/mL. It is preferable that the value be within the above numerical value range since the efficiency of inducing brain vesicles containing neuroepithelial cells is improved.

The first medium can further contain a TGF-β family signal transduction pathway inhibitor. The details of the TGF-β family signal transduction pathway inhibitor are the same as those described above. In a case where SB431542 is used as the TGF-β family signal transduction pathway inhibitor, the final concentration in the medium is preferably 0.5 µM to 10 µM, more preferably 0.75 µM to 5 µM, and further more preferably 1 µM to 3 µM.

Culture of neuroectoderm marker-positive cell aggregates in the first medium is performed for a period required to obtain a telencephalic marker-positive brain organoid. The telencephalic marker is the same as those described above.

For example, in a case where the first medium contains a Wnt signal enhancer and a TGF-β family signal transduction pathway inhibitor, the Wnt signal enhancer is Wnt3a and CHIR99021, and the TGF-β family signal transduction pathway inhibitor is SB431542, the culture period of the neuroectoderm marker-positive cell aggregate in the first medium is preferably 3 to 14 days, more preferably 4 to 12 days, further preferably 5 to 10 days, and particularly preferably 6 days to 8 days.

### <<Step of culturing in a medium substantially not containing extracellular matrix components>>

The production method of the present embodiment can include a step of culturing in a medium substantially not containing extracellular matrix components (hereinafter, also referred to as "second medium"). Specifically, the production method of the present embodiment preferably further includes a step of culturing a cell aggregate obtained by culturing the neuroectoderm marker-positive cell aggregate in the first medium in a medium substantially not containing extracellular matrix components. By having these steps, it is possible to obtain a cell aggregate (brain organoid) containing forebrain marker-positive cells and telencephalic partial tissue marker-positive cells, in addition to the telencephalic marker-positive cells. The culture is preferably performed by suspension culture.

In cell aggregates during suspension culture, cells surface-adhere to each other. In the cell aggregate during suspension culture, a cell-substratum junction is hardly formed with the culture equipment or the like, or even if it is formed, its contribution is small. In some embodiments, in the cell aggregate during suspension culture, an endogenous cell-substratum junction is present inside the aggregate, but a cell-substratum junction is hardly formed with the culture equipment and the like, or even if it is formed, its contribution is small.

The incubator used for the suspension culture is not particularly limited as long as it can perform "suspension culture", and can be appropriately determined by those skilled in the art. Examples of such an incubator include a flask, a tissue culture flask, a culture dish, a petri dish, a tissue culture dish, a multi-dish, a microplate, a microwell plate, a micropore, a multiplate, a multiwell plate, a chamber slide, a petri dish, a tube, a tray, a culture bag, a spinner flask, an Erlenmeyer flask, a roller bottle, and the like.

These incubators are preferably cell non-adhesive to allow suspension culture. As a cell non-adhesive incubator, those in which the surface of the incubator is not artificially treated for the purpose of improving adhesion to cells (for example, coating treatment by extracellular matrix such as basal membrane preparation, laminin, entactin, collagen, and gelatin, or polymer such as polylysine and polyornithine, or surface processing such as positive charge treatment) and the like can be used. As the cell non-adhesive incubator, those in which the surface of the incubator was artificially treated for the purpose of reducing the adhesion to cells (for example, superhydrophilic treatment of MPC polymer, low protein adsorption treatment, and the like) can be used. Stirring may be performed using a spinner flask, a roller bottle, a small bioreactor with stirring blades, and the like. The culture surface of the incubator may have a flat bottom or may have an unevenness.

Suspension culture is preferably performed under high oxygen partial pressure conditions. By suspension culture under high oxygen partial pressure conditions, long-term maintenance culture of the ventricular zone contained in the brain organoid is achieved, and not only can a telencephalic marker-positive cell be formed but also at least a cell aggregate having one or more cells selected from the forebrain marker-positive cells and the telencephalic partial tissue marker-positive cells.

The high oxygen partial pressure condition means an oxygen partial pressure condition that exceeds the oxygen partial pressure (20%) in the air. It is also possible to perform culture at 30% to 60% under high oxygen partial pressure conditions.

The second medium can be prepared using a medium used for culturing animal cells as a basal medium. The basal medium is not particularly limited as long as the basal medium is a medium used to culture animal cells, such as BME medium, BGJb medium, CMRL1066 medium, Glasgow MEM medium, Improved MEM Zinc Option medium, IMDM medium, Medium 199 medium, Eagle MEM medium, αMEM medium, DMEM medium, Ham's medium, Ham's F-12 medium, RPMI1640 medium, Fischer's medium, a mixed medium thereof, and the like.

The second medium may further contain an N2 supplement, chemically defined lipid concentrate, serum, serum substitute, heparin, and the like. The serum substitutes are the same as those described above.

The second medium does not substantially contain extracellular matrix components. Here, "does not substantially contain extracellular matrix components" means that the extracellular matrix components are not intentionally added to the second medium, and it is acceptable that a part or all of the extracellular matrix components added to the first medium be mixed in the second medium, and that the second medium contain extracellular matrix components produced by the brain organoid itself. In one embodiment, the concentration of the extracellular matrix component in the second medium is preferably 1% by volume or less, more preferably 0.5% by volume or less, and further more preferably 0.1% by volume or less, with the combined volume of the second medium and Matrigel as 100% by volume.

Since the second medium contains substantially no extracellular matrix component, it is possible to produce a cell aggregate having not only the telencephalic marker-positive cells but also at least any one or more cells of the forebrain marker-positive cells and the telencephalic partial tissue marker-positive cells.

The culture period in the second medium is preferably 7 to 154 days, more preferably 14 to 147 days, and further more preferably 21 to 140 days. When culture is performed for 77 days or more in the second medium, it tends to be easy to obtain brain organoids having a clear hierarchical structure (inside-out pattern) of the cerebral cortex.

### [Brain organoid]

In a first embodiment, the present invention provides a brain organoid produced by the method for producing a brain organoid of the present embodiment. There may be a difference in the gene expression pattern and the like between the brain organoid of the present embodiment and the brain organoid produced by a production method different from the production method of the brain organoid of the present embodiment. However, it is uncertain whether or not there is such a difference, and in order to specify such a difference and specify the brain organoid of the present embodiment by a gene expression pattern or the like, it is necessary to experience significantly excessive trial and error, and it is substantially impossible. Therefore, it is considered practical to specify the brain organoid of the present embodiment by the above-mentioned production method.

As will be described later in the examples, in the brain organoid of the present embodiment, in a case of performing immunostaining on PAX6, which is a neural stem cell or neural progenitor cell marker, CTIP2, which is a layer V marker of the cerebral cortex, and SATB2, which is a layer II/III marker of the cerebral cortex, an inside-out pattern is observed. In addition, by long-term culture, it can be confirmed by immunostaining that the brain organoid of the present embodiment has S100β or GFAP-positive cells, which are astrocyte markers.

### [Method and kit for evaluating toxicity and drug efficacy of test substance]

In a first embodiment, the present invention provides a method for evaluating the toxicity and drug efficacy of a test substance, which includes contacting a test substance with the brain organoid of the present embodiment and testing the effect of the test substance on the brain organoid.

According to the method of the present embodiment, it is possible to evaluate the drug efficacy of the test substance on the brain with good reproducibility. In addition, by using human-derived brain organoids, it becomes possible to accurately evaluate the drug efficacy of the test substance in humans.

Examples of the test substance include a natural compound library, a synthetic compound library, an existing drug library, and the like.

Methods for testing an effect of a test substance on a brain organoid include diameter measurement, morphological analysis, cell viability analysis, gene expression pattern analysis, immunostaining of sections, measurement of nerve activity, and the like, in the presence and absence of the test substance.

### [Therapeutic agents and therapeutic pharmaceutical compositions for diseases based on disorders of nervous system cells or nervous tissues]

The brain organoid of the present embodiment can be used as a therapeutic agent for treating a disease by transplanting it into the brain of a patient with a disease based on a disorder of nervous system cells or nervous tissues such as Alzheimer's disease, microcephaly, and autism.

In addition, the therapeutic agent of the present embodiment contains the brain organoid of the present embodiment as an effective component, further includes a buffer solution for suspending the brain organoid, an existing therapeutic agent for a disease based on a disorder of nervous system cells or nervous tissue, and the like, and may be prepared in the form of a therapeutic pharmaceutical composition. Here, "containing as an effective component" means that a brain organoid is contained in an amount sufficient to obtain a therapeutic effect on a disease based on a disorder of nervous system cells or nervous tissues, and the content thereof is not particularly limited.

### Examples

Subsequently, the present invention will be described in more detail with reference to examples, but the present invention is not limited thereto.

### [Experimental Example 1]

Human iPS cells (PChiPS771 strain, Lot. A01QM28, manufactured by ReproCELL Inc.) were subjected to feeder-free culture according to the method described in "Nakagawa M., et al., A novel efficient feeder-free culture system for the derivation of human induced pluripotent stem cells, Scientific Reports, 4, 3594, 2014". StemFit AK02N (manufactured by Ajinomoto Co., Inc.) was used as a feeder-free medium, and iMatrix-511 (manufactured by Nippi Inc.) was used as a feeder-free scaffold.

As a specific expansion culture operation, first, human iPS cells (PChiPS771 strain, Lot.A01QM28, manufactured by ReproCELL Inc.), which have become 60% to 80% confluent (60% to 80% of the culture area is covered with cells), were washed with phosphate-buffered saline (hereinafter, abbreviated as "PBS"), the cells were dispersed in a single cell using TrypLE Select (manufactured by Thermo Fisher Scientific Inc.). After that, the human iPS cells dispersed in the single cells were seeded in a plastic culture dish coated with iMatrix-511 (manufactured by Nippi), Y27632 (ROCK inhibitor, final concentration 10 µM) was added, and the cells were subjected to feeder-free culture on a StemFit AK02N medium. As the plastic culture dish, a 60 mm dish (manufactured by IWAKI&CO. LTD., for cell culture) was used, and the number of seeded cells of human iPS cells dispersed in the single cells was 3 × 10⁴.

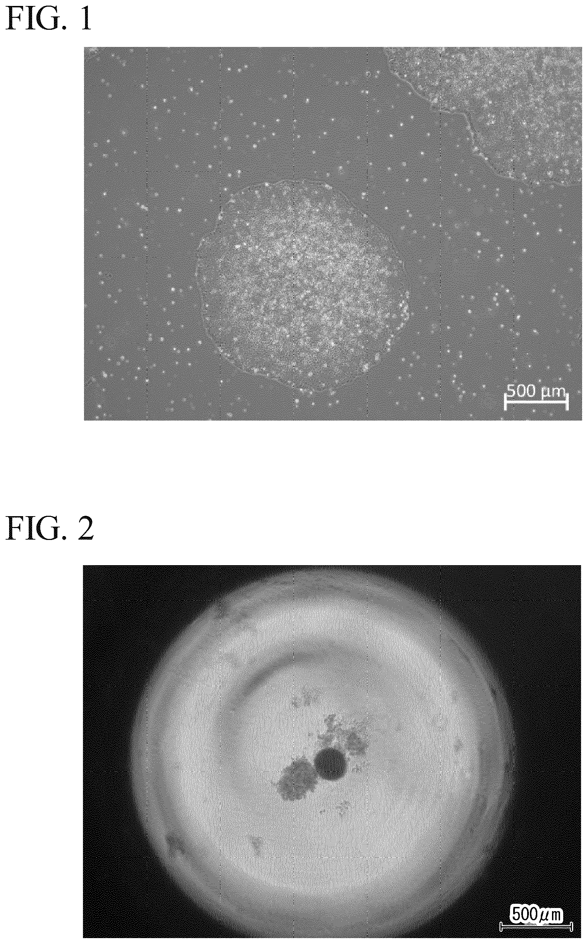

One day after seeding the cells, the medium was changed to StemFit AK02N medium not containing Y27632. After that, the medium was changed once every 1 to 2 days with StemFit AK02N medium not containing Y27632. After that, 6 days after seeding the cells, the cells became 80% confluent. Fig. 1 shows a photomicrograph of iPS cells observed in a bright field 7 days after seeding.

### [Experimental Example 2]

80% confluent human iPS cells (PChiPS771 strain, Lot. A01QM28, manufactured by ReproCELL Inc.) were treated in the presence of Y27632 (ROCK inhibitor, 10 µM) for 2 hours to obtain human iPS cells.

The human iPS cells treated for 2 hours were subjected to single cell treatment by a pipetting operation using a cell dispersion (product name "TrypLE Select", manufactured by Thermo Fisher Scientific Inc.). The human iPS cells made into single cells were subjected to suspension culture in 100 µL of an aggregation medium, at 37°C in the presence of 5% by volume CO₂ in a container so as to be 2 × 10⁴ cells per well of a non-cell adhesive 96-well culture plate (product name "PrimeSurface 96V bottom plate", manufactured by Sumitomo Bakelite Co., Ltd.).

As the aggregation medium, a medium in which Non-essential Amino Acids (manufactured by Thermo Fisher Scientific Inc., dilution concentration 200 times), Penicillin/Streptomycin (manufactured by NACALAI TESQUE, INC., dilution concentration 100 times), Glutamax (manufactured by Thermo Fisher Scientific Inc., dilution concentration 100 times), 1 × 2-Mercaptoethanol (manufactured by Thermo Fisher Scientific Inc., dilution concentration 1,000 times), Dorsomorphin (manufactured by Sigma, final concentration 2 µM), and A-83-01 (final concentration 2 µM) were added to StemFit AK02N (manufactured by Ajinomoto Co., Ltd.) was used.

At the start of suspension culture (0th day of culture, hereinafter, unless otherwise specified, the number of culture days is represented by the number of culture days from the start of suspension culture), Y27632 (final concentration 30 µM) was added to the aggregation medium. In addition, Further, on the 1st day after the start of the suspension culture, 150 µL of a medium containing Y27632 (final concentration 10 µM) was added to a medium having the same composition as the aggregation medium, and the suspension culture was continued without changing the medium until the 7th day after the start of the suspension culture. Fig. 2 is a photomicrograph showing the results of bright-field observation of cell aggregates on the 7th day after the start of suspension culture with an inverted microscope (Olympus Corporation).

The cell aggregate on the 7th day after the start of suspension culture was subjected to immunostaining to examine the expression of neuroectoderm markers. Specifically, the cell aggregate was fixed with a 4 mass% paraformaldehyde aqueous solution, replaced with a sucrose solution, and a frozen section was prepared using Leica CM3050 S (manufactured by Leica Camera AG). Subsequently, these frozen sections were subjected to immunostaining with neuroectoderm markers.

As a neuroectoderm marker, βIII-TUBULIN was examined. As a result, it was clarified that the cell aggregate on the 7th day after the start of suspension culture was neuroectoderm marker-positive.

### [Experimental Example 3]

On the 7th day after the start of suspension culture in Experimental Example 2, 230 µL of the aggregation medium was removed from each well. Subsequently, 150 µL/well of the first medium was added, mixed on an ice bath so that the extracellular matrix and other components were uniformly dispersed, and subjected to suspension culture at 37°C in the presence of 5% by volume CO² in the container without stirring.

As the first medium, Dulbecco's Modified Eagle Medium: Nutrient Mixture F-12 (manufactured by Thermo Fisher Scientific Inc.) was added to 1 × N2 Supplement (manufactured by Thermo Fisher Scientific Inc., dilution concentration 200 times), Heparin Sodium Salt (manufactured by Sigma, final concentration 10 µg / mL), Non-essential Amino Acids (manufactured by Thermo Fisher Scientific Inc., dilution concentration 200 times), Penicillin/ Streptomycin (manufactured by NACALAI TESQUE INC., dilution concentration 100 times), Glutamax (Thermo Fisher Scientific Inc., dilution concentration 100 times), Wnt-3a (Human, Recombinant, manufactured by R&D Systems Inc., final concentration 4 ng/mL), CHIR99021 (manufactured by Axon Medchem, final concentration 1 µM), and SB-431542 (manufactured by Sigma, final concentration 1 µM), and 2% by volume, 10% by volume, 30% by volume, or 50% by volume of Matrigel (manufactured by Corning Inc.) was further added to prepare various first media having different extracellular matrix concentrations.

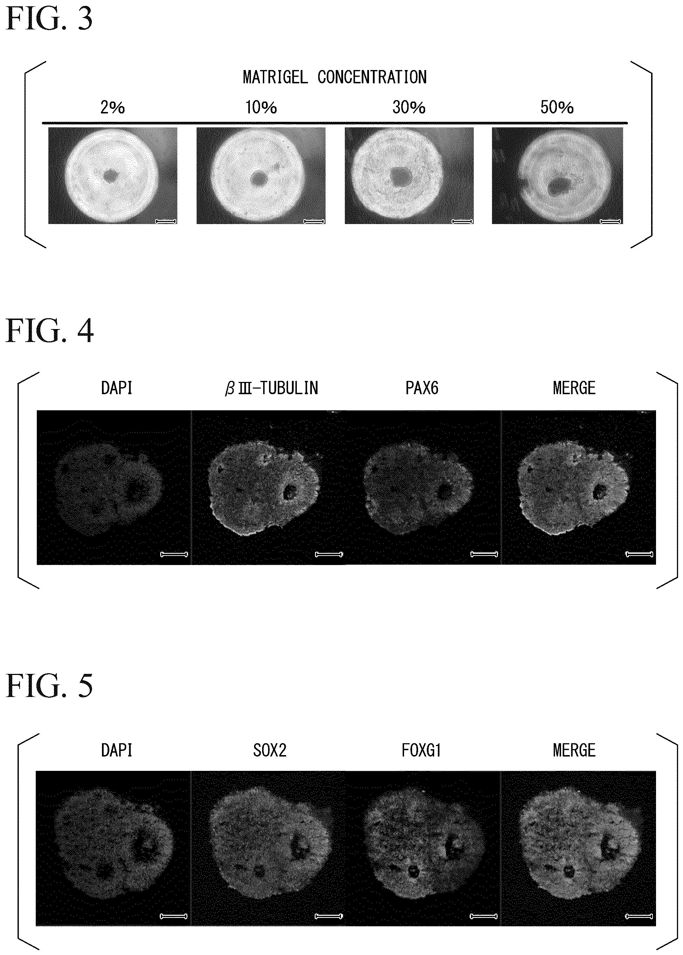

Fig. 3 is a photomicrograph showing the results of bright-field observation of each cell aggregate on the 14th day after the start of suspension culture in Experimental Example 3.

Subsequently, frozen sections were prepared in the same manner as in Experimental Example 2 for each cell aggregate on the 14th day after the start of suspension culture in Experimental Example 3, subjected to immunostaining, and brain markers were evaluated.

Specifically, expression of FOXG1 (anti-FOXGl antibody, ABCAM, rabbit), which is a forebrain and telencephalic marker, βIII-TUBULIN (anti-βIII-TUBULIN antibody, SIGMA, mouse), which is a juvenile neural cell marker, and PAX6 (anti-PAX6 antibody, MBL, rabbit), which is a neural stem cell or neural progenitor cell marker, was examined. Each sample after immunostaining was observed with a fluorescence microscope using a confocal microscope (manufactured by ZEISS).

Figs. 4 and 5 are fluorescence photomicrographs showing the results of immunostaining of cell aggregates cultured at a Matrigel concentration of 30% by volume. In Fig. 4, "DAPI" indicates the result of staining the frozen section of the cell aggregate with DAPI, "βIII-TUBULIN" is the result of staining the frozen section of the cell aggregate with the anti-βIII-TUBULIN antibody, "PAX6" indicates the result of staining frozen sections of cell aggregates with an anti-PAX6 antibody, and "MERGE" indicates the result of synthesizing the above results.

In Fig. 5, "DAPI" and "MERGE" have the same meanings as those in Fig. 4, "SOX2" indicates that it is the result of staining a frozen section of a cell aggregate with an anti-SOX2 antibody, and "FOXG1" indicates the result of staining a frozen section of a cell aggregate with anti-FOXG1 antibody.

As a result, it was clarified that the cell aggregate cultured at a Matrigel concentration of 30% by volume (14th day after the start of suspension culture of Experimental Example 3) was positive for FOXG1, βIII-TUBULIN, and PAX6, and was at least a forebrain and telencephalic marker-positive brain organoid.

### [Experimental Example 4]

The same medium containing Matrigel at a concentration of 50% by volume (first medium of Experimental Example 4) as that in Experimental Example 3 except that StemFit AK02N (manufactured by Ajinomoto Co., Ltd.) was used instead of Dulbecco's Modified Eagle Medium: Nutrient Mixture F-12 (manufactured by Thermo Fisher Scientific Inc.) in the first medium of Experimental Example 3 was prepared, and the cell aggregate on the 7th day after the start of suspension culture of Experimental Example 2 was cultured.

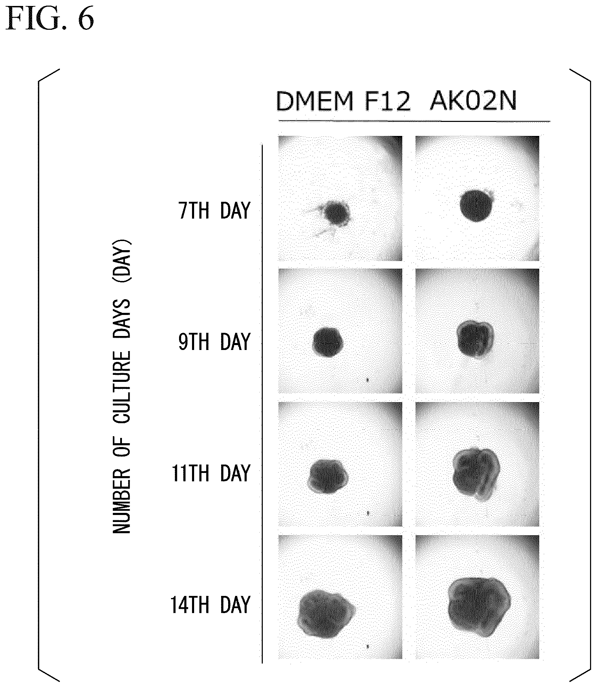

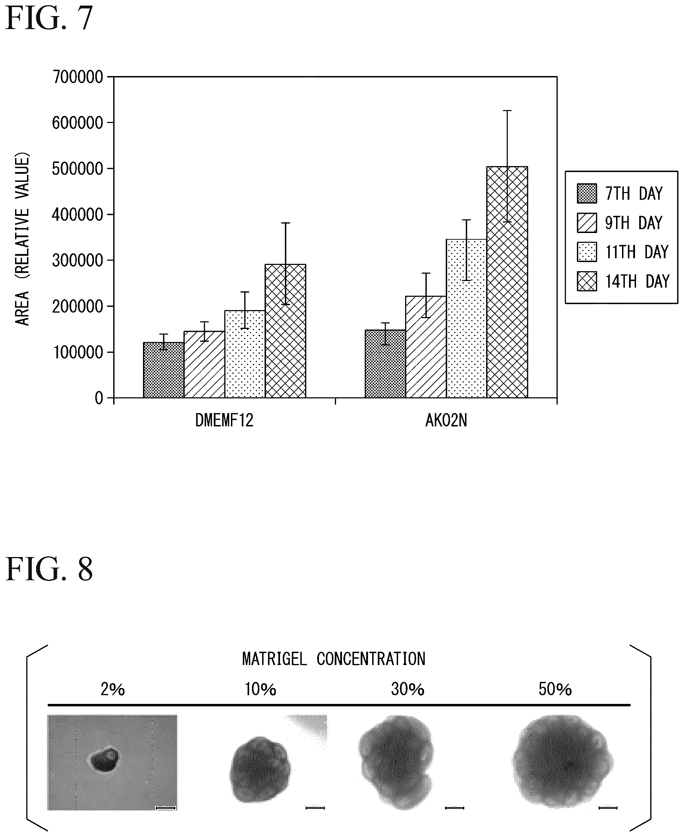

Fig. 6 is a photomicrograph showing the result of bright-field observation of each cell aggregate on the 7th, 9th, 11th, and 14th days after the start of suspension culture. Fig. 7 is a graph showing the result of measuring a two-dimensional projected area of each cell aggregate from the photograph of Fig. 6.

In Figs. 6 and 7, "DMEM F12" is the result of using the first medium with a Matrigel concentration of 50% by volume of Experimental Example 3, and "AK02N" is the result of using the first medium of Experimental Example 4.

Similar to Experimental Example 3, for the cell aggregate cultured using the first medium of Experimental Example 4, a frozen section was prepared, similar to Experimental Example 3, and subjected to immunostaining to evaluate the brain marker. As a result, it was clarified that the cell aggregate cultured using the first medium of Experimental Example 4 was also positive for FOXG1, βIII-TUBULIN, and PAX6, and was at least a forebrain and telencephalic marker-positive brain organoid.

### [Experimental Example 5]

In Experimental Example 3, each cell aggregate on the 14th day of culture cultured in various first media having different extracellular matrix concentrations was collected from each well, and transferred to a 50 mL Falcon (trademark) conical tube (manufactured by Corning Inc.) containing 10 mL of PBS. Subsequently, the Matrigel (extracellular matrix) was removed by performing 5 rounds of inversions and admixture and removing the supernatant.

Subsequently, each cell aggregate was collected from a 50 mL Falcon (trademark) conical tube, and transferred to a 30 mL single-use bioreactor. Subsequently, 20 mL of the second medium was added, and suspension culture was started while stirring. The stirring speed was set to 50 rpm, and the medium was changed once every 3 to 4 days.

As a second medium, a medium not containing an extracellular matrix was used. Specifically, as the second medium, one in which Dulbecco's Modified Eagle Medium: Nutrient Mixture F-12 (manufactured by Thermo Fisher Scientific Inc.) was added to 1 × N2 Supplement (manufactured by Thermo Fisher Scientific Inc., dilution concentration 200 times), B-27 Supplement (manufactured by Thermo Fisher Scientific Inc., dilution concentration 100 times), 1 × Non-essential Amino Acids (manufactured by Thermo Fisher Scientific Inc., dilution concentration 200 times), 1 × Penicillin/Streptomycin (manufactured by NACALAI TESQUE INC., dilution concentration 100 times), 1 × 2-Mercaptoethanol (manufactured by Thermo Fisher Scientific Inc., dilution concentration 1,000 times), and Insulin Solution (Human, recombinant, manufactured by FUJIFILM Wako Pure Chemical Corporation, 2.5 µg/mL) was used.

For each cell aggregate on the 40th day and the 70th day after the start of suspension culture, similar to Experimental Example 3, a frozen section was prepared, and subjected to immunostaining to evaluate a brain marker. Specifically, PAX6 (anti-PAX6 antibody, sheep), which is a neural stem cell or neural progenitor cell marker, CTIP2 (anti-CTIP2 antibody, ABCAM, rat), which is a layer V marker of the cerebral cortex, and SATB2 (anti-SATB2 antibody, mouse), which is layer II/III marker of the cerebral cortex, were subjected to immunostaining, and observed with a fluorescence microscope using a confocal microscope (manufactured by ZEISS).

Fig. 8 is a photomicrograph showing the result of bright-field observation of each of cell aggregates having different Matrigel concentrations on the 40th day after the start of suspension culture. As a result, it was clarified that as the concentration of Matrigel increases, the size of the cell aggregate increases. In addition, it was clarified that in a case where the Matrigel concentration is 10% by volume or more, an aggregate having a neural tube-like structure can be formed.

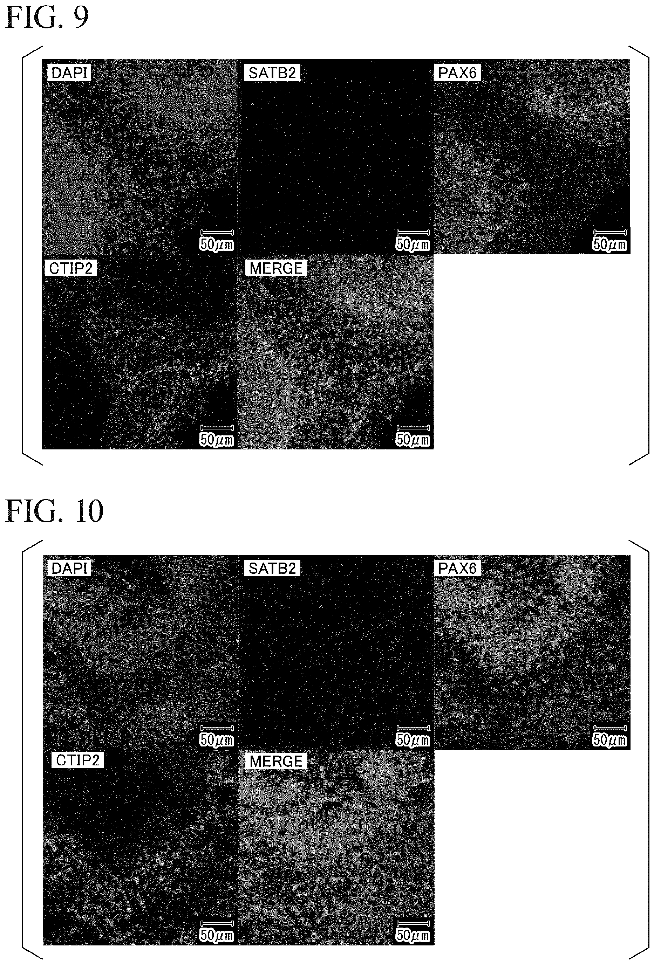

Fig. 9 is a fluorescence photomicrograph showing results of preparing a frozen section from a cell aggregate obtained using a first medium with a Matrigel concentration of 50% by volume, immunostaining, and evaluating a brain marker on the 40th day after the start of suspension culture. As a result, it was clarified that the cell aggregate obtained on the 40th day after the start of suspension culture has a neural tube-like structure and has a region having a high PAX6-positive cell density, which is a neural stem cell and neural progenitor cell marker. In addition, although CTIP2-positive cells, which are layer V markers of the cerebral cortex, were disposed outside the region having a high PAX6-positive cell density, SATB2-positive cells, which are layer II/III markers of the cerebral cortex, were not observed. From the above results, it was clarified that the obtained cell aggregates were at least neural stem cells or neural progenitor cells and layer V marker-positive brain organoids of the cerebral cortex.

In Fig. 9, "DAPI" indicates the result of staining with DAPI, "SATB2" indicates the result of staining with the anti-SATB2 antibody, "PAX6" indicates the result of staining with the anti-PAX6 antibody, "CTIP2" indicates the result of staining with an anti-CTIP2 antibody, and "MERGE" indicates the result of synthesizing the above results. Hereinafter, the same applies to Figs. 10 to 15.

Fig. 10 is a fluorescence photomicrograph showing the results of preparing a frozen section from a cell aggregate obtained using a first medium with a Matrigel concentration of 30% by volume, immunostaining, and evaluating a brain marker on the 40th day after the start of suspension culture. As a result, it was clarified that the cell aggregate obtained on the 40th day after the start of suspension culture has a neural tube-like structure and has a region having a high PAX6-positive cell density, which is a neural stem cell and neural progenitor cell marker. In addition, although CTIP2-positive cells, which are layer V markers of the cerebral cortex, are disposed outside the region having a high PAX6-positive cell density, SATB2-positive cells, which are layer II/III markers of the cerebral cortex, were not observed. From the above results, it was clarified that the obtained cell aggregates were at least neural stem cells or neural progenitor cells and layer V marker-positive brain organoids of the cerebral cortex.

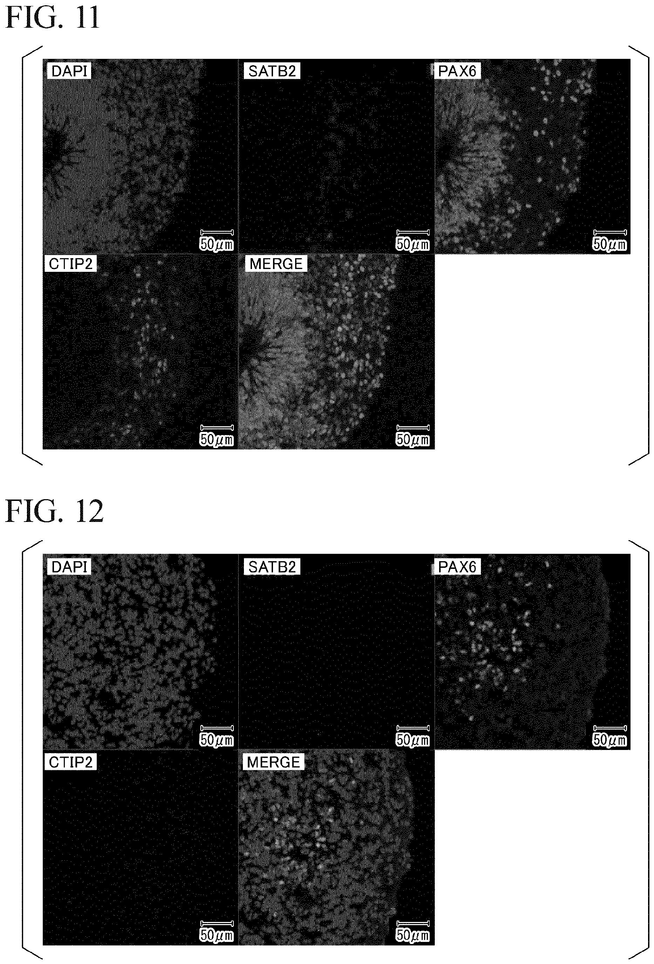

Fig. 11 is a fluorescence photomicrograph showing results of preparing a frozen section from a cell aggregate obtained using a first medium with a Matrigel concentration of 10% by volume, immunostaining, and evaluating a brain marker on the 40th day after the start of suspension culture. As a result, it was clarified that the cell aggregate obtained on the 40th day after the start of suspension culture has a neural tube-like structure and has a region having a high PAX6-positive cell density, which is a neural stem cell and neural progenitor cell marker. In addition, although CTIP2-positive cells, which are layer V markers of the cerebral cortex, were disposed outside the region having a high PAX6-positive cell density, SATB2-positive cells, which are layer II/III markers of the cerebral cortex, were not observed. From the above results, it was clarified that the obtained cell aggregates were at least neural stem cells or neural progenitor cells and layer V marker-positive brain organoids of the cerebral cortex.

Fig. 12 is a fluorescence photomicrograph showing results of preparing a frozen section from a cell aggregate obtained using a first medium with a Matrigel concentration of 2% by volume, immunostaining, and evaluating a brain marker on the 40th day after the start of suspension culture. As a result, the number of cell aggregates obtained on the 40th day after the start of suspension culture was smaller than that of cell aggregates having PAX6-positive cells, which are neural stem cell or neural progenitor cell markers with a Matrigel concentration of 10% to 50% by volume. In addition, CTIP2-positive cells, which are layer V markers of the cerebral cortex, and SATB2-positive cells, which are layer II/III markers of the cerebral cortex, were not observed. From the above results, it was clarified that the obtained cell aggregates were at least neural stem cells or neural progenitor cells and layer V marker-positive brain organoids of the cerebral cortex.

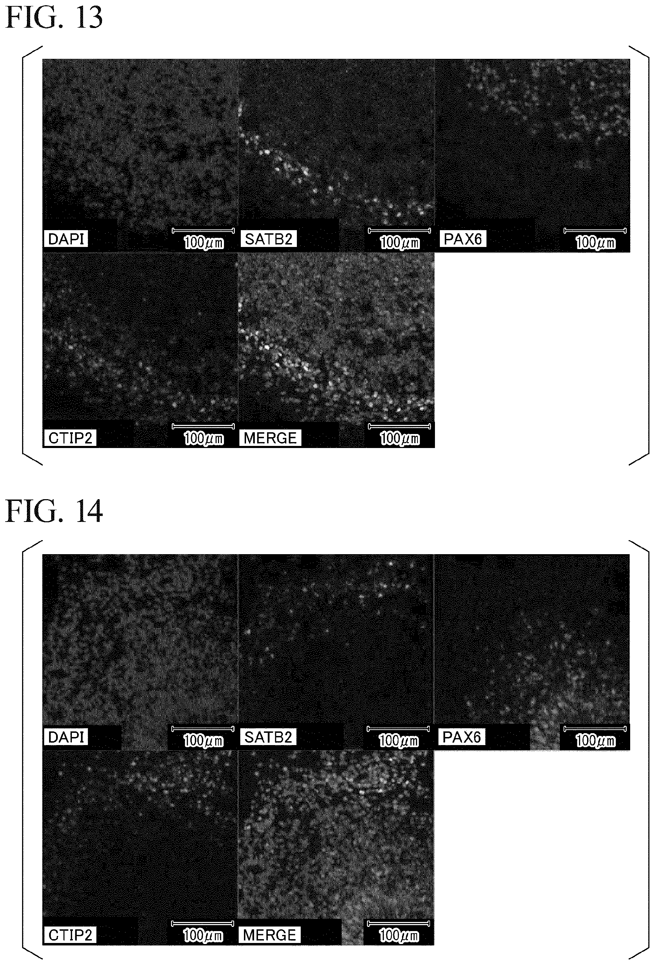

Fig. 13 is a fluorescence photomicrograph showing results of preparing a frozen section from a cell aggregate obtained using a first medium with a Matrigel concentration of 50% by volume, immunostaining, and evaluating a brain marker on the 70th day after the start of suspension culture. As a result, it was clarified that the cell aggregate obtained on the 70th day after the start of suspension culture has a neural tube-like structure and has a region having a high PAX6-positive cell density, which is a neural stem cell or neural progenitor cell marker. In addition, it was shown that a CTIP2-positive cell layer, which is a layer V marker of the cerebral cortex, was present outside the region having a high PAX6-positive cell density, SATB2-positive cells, which are layer II/III markers of the cerebral cortex, were disposed further outside, and a cerebral cortex-like hierarchical structure was formed. From the above results, it was clarified that the obtained cell aggregates were at least neural stem cells or neural progenitor cells, a layer V of the cerebral cortex, and a layer II/III marker-positive brain organoid of the cerebral cortex.

Fig. 14 is a fluorescence photomicrograph showing results of preparing a frozen section from a cell aggregate obtained using a first medium with a Matrigel concentration of 30% by volume, immunostaining, and evaluating a brain marker on the 70th day after the start of suspension culture. As a result, it was clarified that the cell aggregate obtained on the 70th day after the start of suspension culture has a neural tube-like structure and has a region having a high PAX6-positive cell density, which is a neural stem cell or neural progenitor cell marker. In addition, it was shown that a CTIP2-positive cell layer, which is a layer V marker of the cerebral cortex, was present outside the region having a high PAX6-positive cell density, SATB2-positive cells, which are layer II/III markers of the cerebral cortex, were disposed further outside, and a cerebral cortex-like hierarchical structure was formed. From the above results, it was clarified that the obtained cell aggregates were at least neural stem cells or neural progenitor cells, a layer V of the cerebral cortex, and a layer II/III marker-positive brain organoid of the cerebral cortex.

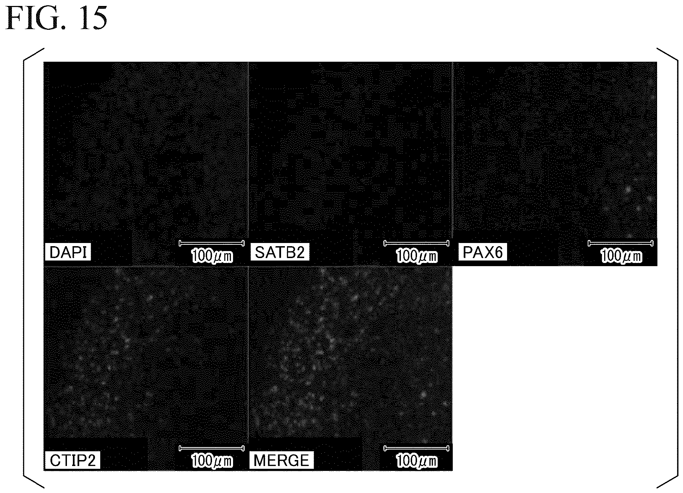

Fig. 15 is a fluorescence photomicrograph showing results of preparing a frozen section from a cell aggregate obtained using a first medium with a Matrigel concentration of 10% by volume, immunostaining, and evaluating a brain marker on the 70th day after the start of suspension culture. As a result, it was clarified that the cell aggregate obtained on the 70th day after the start of suspension culture has a neural tube-like structure and has a region having a high PAX6-positive cell density, which is a neural stem cell or neural progenitor cell marker. In addition, CTIP2-positive cells, which are layer V markers of the cerebral cortex, were disposed outside the region having a high PAX6-positive cell density. On the other hand, SATB2-positive cells, which are layer II/III markers of the cerebral cortex, were hardly observed. From the above results, it was clarified that the obtained cell aggregates were at least neural stem cells or neural progenitor cells and layer V marker-positive brain organoids of the cerebral cortex.

### Industrial Applicability

According to the present invention, it is possible to provide a method for producing a telencephalic marker-positive brain organoid.

## Claims

1. A method for producing a brain organoid, comprising:
a step of culturing a neuroectoderm marker-positive cell aggregate in a medium containing an extracellular matrix with a concentration of more than 10% by volume.

2. The method for producing a brain organoid according to Claim 1, wherein a concentration of the extracellular matrix in the medium is 20% by volume to 50% by volume.

3. The method for producing a brain organoid according to Claim 1 or 2,
wherein the medium further contains a Wnt signal enhancer.

4. The method for producing a brain organoid according to any one of Claims 1 to 3,
wherein the medium further contains a transforming growth factor β family signal transduction pathway inhibitor.

5. The method for producing a brain organoid according to any one of Claims 1 to 4,
wherein the step is a step of culturing the neuroectoderm marker-positive cell aggregate in a medium containing an extracellular matrix with a concentration of more than 10% by volume in a dispersed state without inserting the cell aggregate into the extracellular matrix.

6. The method for producing a brain organoid according to any one of Claims 1 to 5, further comprising:
a step of performing culture in a medium substantially not containing an extracellular matrix after the step.

7. The method for producing a brain organoid according to Claim 6,
wherein the step of performing culture in a medium substantially not containing an extracellular matrix is performed by suspension culture.

8. The method for producing a brain organoid according to Claim 6 or 7,
wherein the step of performing culture in a medium substantially not containing an extracellular matrix is performed under high oxygen partial pressure conditions.

9. The method for producing a brain organoid according to any one of Claims 6 to 8,
wherein the step of performing culture in a medium substantially not containing an extracellular matrix is performed while stirring.

10. The method for producing a brain organoid according to any one of Claims 1 to 9,
wherein the neuroectoderm marker-positive cell aggregate is derived from a human.

11. A brain organoid produced by the production method according to any one of Claims 1 to 10.

12. The brain organoid according to Claim 11, comprising:
at least a telencephalic marker-positive cell.

13. The brain organoid according to Claim 12, further comprising:
a telencephalic partial tissue marker-positive cell.

14. The brain organoid according to Claim 13,
wherein the telencephalic partial tissue marker-positive cell is at least one selected from the group consisting of a cerebral cortex, a basal ganglia, a hippocampus, and a choroid plexus.

15. A kit for evaluating drug efficacy of a test substance, comprising: the brain organoid according to any one of Claims 11 to 14.

16. A method for evaluating drug efficacy of a test substance, the method comprising:
a step of contacting the test substance with the brain organoid according to any one of Claims 11 to 14; and
a step of testing an effect of the test substance on the brain organoid.

17. A therapeutic agent for a disease based on a disorder of a nervous system cell or a nervous tissue, the therapeutic agent comprising: the brain organoid according to any one of Claims 11 to 14.

18. A pharmaceutical composition for treating a disease based on a disorder of a nervous system cell or a nervous tissue, the pharmaceutical composition comprising:
the brain organoid according to any one of Claims 11 to 14 as an effective component.
